# EUROPEAN PATENT SPECIFICATION

(11) **EP 3 756 637 B1**
(45) Date of publication and mention of the grant of the patent: **15.02.2023**
(21) Application number: 19197348.6
(22) Date of filing: 13.09.2019
(51) Int. Cl.: A61H 1/02, A61H 3/00

(54) **BALANCE ASSISTANCE SYSTEM AND WEARABLE DEVICE**
GLEICHGEWICHTUNTERSTÜTZUNGSSYSTEM UND TRAGBARE VORRICHTUNG
SYSTÈME D'AIDE À L'ÉQUILIBRE ET DISPOSITIF PORTABLE

(30) Priority: 26.06.2019 TW 108122318
(43) Date of publication of application: 30.12.2020
(73) Proprietor: Wistron Corporation, Hsichih, 22181 New Taipei City (TW)
(72) Inventor: Chan, Hsiang-Min, 22181 New Taipei City (TW); Chang, Yao-Tsung, 22181 New Taipei City (TW); Kao, Chuan-Yen, 22181 New Taipei City (TW); Chen, Yin-Yu, 22181 New Taipei City (TW); Tsou, Tsung-Yin, 22181 New Taipei City (TW)
(74) Representative: Straus, Alexander

(56) References cited:
- EP-A1- 2 514 400
- WO-A1-2017/146571
- CN-A- 108 436 916
- DE-A1-102015 215 524
- US-A1- 2014 260 714

## Description

### Field of the Invention

The present invention relates to a balance assistance system and a wearable device, particularly a balance assistance system capable of assisting a user in balance to prevent the user from falling down and a wearable device equipped with the balance assistance system.

### Background of the Invention

Due to the coming of aging society and lack of medical resource, exoskeleton becomes a major topic for robotic researchers. At present, the framework of the exoskeleton essentially consists of a plurality of motors and reducers. The exoskeleton detects a motion of a user by various sensors and controls the corresponding motor to adjust a joint angle and a posture of the user. Since the conventional exoskeleton is not equipped with a balance device, the user needs to balance by himself/herself or a cane. Once the user loses balance or gets hit, the user may fall down and be hurt.

Furthermore, the related art China Patent Publication No. 108436916 (hereinafter `916 art) provides a gyro driver based balance device and control method thereof. However, `916 art only adjusts the rotating speed of the flywheel according to the tilt angle, but `916 art does not further adjust the rotating speed of the flywheel according to different behavior modes. Thus, `916 art cannot provide different balance assistance for the user according to different behavior modes.

### Summary of the Invention

The present invention aims at providing a balance assistance system capable of assisting a user in balance to prevent the user from falling down and a wearable device equipped with the balance assistance system, thereby resolving the aforesaid problems.

This is achieved by a balance assistance system according to claim 1 and a wearable device according to claim 11. The dependent claims pertain to corresponding further developments and improvements.

As will be seen more clearly from the detailed description following below, the claimed balance assistance system includes a rotating member, a first driving unit, a sensing module, and a processing unit. The first driving unit is connected to the rotating member. The first driving unit is configured to drive the rotating member to rotate. The processing unit is electrically connected to the first driving unit and the sensing module. The processing unit receives a sensing signal from the sensing module. The processing unit determines a current behavior mode from a plurality of behavior modes according to the sensing signal. The processing unit controls the first driving unit to adjust a rotating speed of the rotating member according to the current behavior mode.

As will be seen more clearly from the detailed description following below, the claimed wearable device includes a wearable object and a balance assistance system. The balance assistance system is disposed on the wearable object. The balance assistance system includes a rotating member, a first driving unit, a sensing module, and a processing unit. The first driving unit is connected to the rotating member. The first driving unit is configured to drive the rotating member to rotate. The processing unit is electrically connected to the first driving unit and the sensing module. The processing unit receives a sensing signal from the sensing module. The processing unit determines a current behavior mode from a plurality of behavior modes according to the sensing signal. The processing unit controls the first driving unit to adjust a rotating speed of the rotating member according to the current behavior mode.

### Brief Description of the Drawings

In the following, the invention is further illustrated by way of example, taking reference to the accompanying drawings thereof:
FIG. 1 is a perspective view illustrating a wearable device according to an embodiment of the disclosure,
FIG. 2 is a perspective view illustrating the wearable device shown in FIG. 1 from another viewing angle,
FIG. 3 is a functional block diagram illustrating a balance assistance system shown in FIG. 1,
FIG. 4 is a flowchart illustrating a control manner of the balance assistance system shown in FIG. 3,
FIG. 5 is a front view illustrating a wearable device according to another embodiment of the disclosure,
FIG. 6 is a perspective view illustrating the balance assistance system shown in FIG. 5 after rotating, and
FIG. 7 is a functional block diagram illustrating the balance assistance system shown in FIG. 5.

### Detailed Description

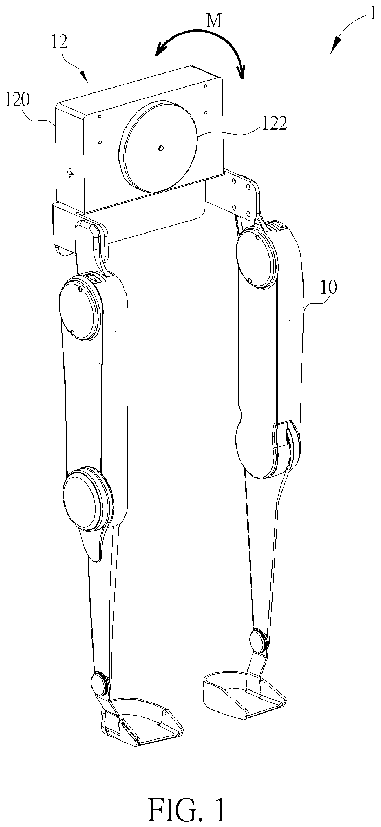

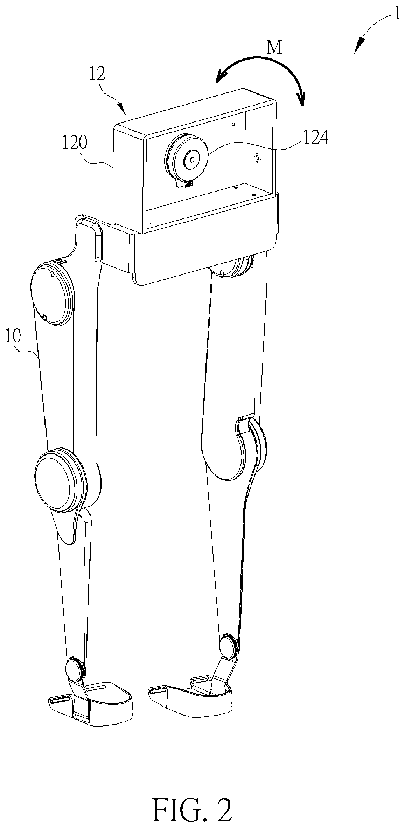

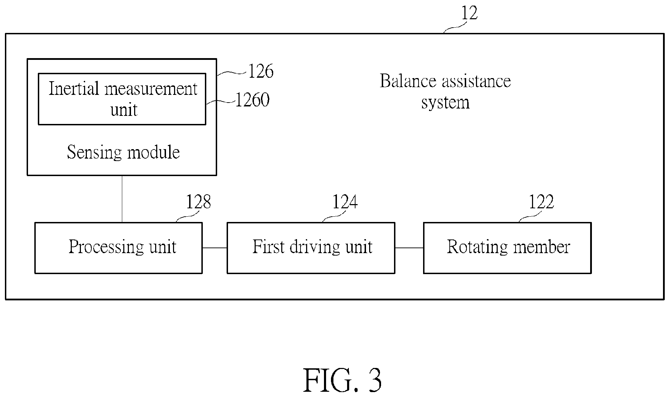

As shown in FIGs. 1 to 3, a wearable device 1 includes a wearable object 10 and a balance assistance system 12, wherein the balance assistance system 12 is disposed on the wearable object 10. It should be noted that the balance assistance system 12 may be disposed on any position of the wearable object 10 (e.g. waist, sole, etc.) according to practical applications, so the disclosure is not limited to the embodiment shown in the figure. In this embodiment, the wearable object 10 may be, but not limited to, an exoskeleton. Furthermore, the balance assistance system 12 of this embodiment disposed on the wearable object 10 is for illustration purpose. However, in another embodiment, the balance assistance system 12 may also be disposed on a movable robot or other movable objects according to practical applications.

The wearable object 10 may be worn by a user and the balance assistance system 12 may assist the user in balance to prevent the user from falling down. The balance assistance system 12 includes a casing 120, a rotating member 122, a first driving unit 124, a sensing module 126, and a processing unit 128. The rotating member 122 is disposed on the casing 120, wherein a rotating shaft of the rotating member 122 may be parallel or perpendicular to a desired direction for balance according to practical applications. The first driving unit 124 is disposed in the casing 120 and connected to the rotating member 122. The first driving unit 124 is configured to drive the rotating member 122 to rotate. The processing unit 128 is electrically connected to the first driving unit 124 and the sensing module 126. In this embodiment, the rotating member 122 may be a flywheel or the like, the first driving unit 124 may be a motor, and the processing unit 128 may be a processor or a controller with signal processing function.

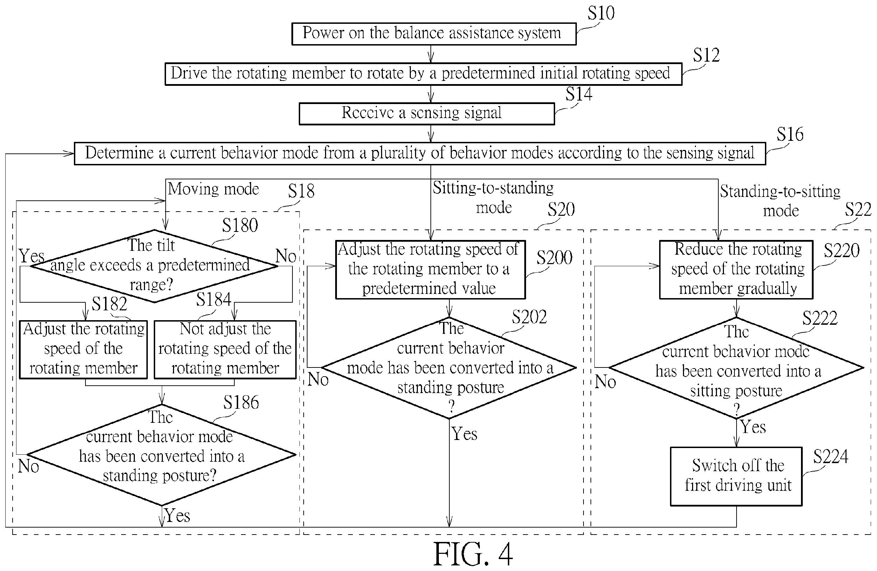

In this embodiment, when the balance assistance system 12 is powered on (step S10 in FIG. 4), the processing unit 128 may control the first driving unit 124 to drive the rotating member 122 to rotate by a predetermined initial rotating speed (step S12 in FIG. 4), so as to speed up reaction of the rotating member 122 for follow-up motion of the user and reduce reaction time of the rotating member 122. When the rotating member 122 rotates, the rotating member 122 generates a moment of inertia M. The predetermined initial rotating speed mentioned in the above may be determined according to practical applications. It should be noted that, in another embodiment, the step S12 in FIG. 4 may be omitted according to practical applications.

Then, the processing unit 128 receives a sensing signal from the sensing module 126 (step S14 in FIG. 4). Then, the processing unit 128 determines a current behavior mode from a plurality of behavior modes according to the sensing signal (step S16 in FIG. 4). In this embodiment, the plurality of behavior modes may include a moving mode, a sitting-to-standing mode, and a standing-to-sitting mode, but is not so limited. The behavior mode may be set according to practical applications. Furthermore, the sensing module 126 may include an inertial measurement unit (IMU) 1260, wherein the inertial measurement unit 1260 is configured to sense a tilt angle of the user.

In an embodiment of the disclosure, the sensing module 126 may further include a plurality of buttons (not shown) corresponding to the plurality of behavior modes. When the user presses one of the buttons, the button outputs the aforesaid sensing signal. At this time, the processing unit 128 may determine the current behavior mode from the plurality of behavior modes according to the sensing signal corresponding to the button.

In another embodiment of the disclosure, the sensing module 126 may further include a skin sensor, a force sensor, or a displacement sensor (not shown). At this time, the processing unit 128 may determine the current behavior mode of the user according to the sensing signal of the skin sensor, the force sensor, or the displacement sensor. It should be noted that the principle of the skin sensor, the force sensor, or the displacement sensor is well known by one skilled in the art, so it will not be depicted herein in detail.

After determining the current behavior mode, the processing unit 128 controls the first driving unit 124 to adjust a rotating speed of the rotating member 122 according to the current behavior mode (steps S18, S20, and S22 in FIG. 4).

When the current behavior mode is the moving mode (Step S18 in FIG. 4), the body of the user may naturally swing back and forth, such that the inertial measurement unit 1260 senses a tilt angle of the user. At this time, the processing unit 128 further determines whether the tilt angle sensed by the inertial measurement unit 1260 exceeds a predetermined range (step S180 in FIG. 4). When the processing unit 128 determines that the tilt angle of the user exceeds the predetermined range, the processing unit 128 controls the first driving unit 124 to adjust the rotating speed of the rotating member 122 according to a variation of the tilt angle of the user (step S182 in FIG. 4). In this embodiment, the tilt angle of the user may include a forward tilt angle and a backward tilt angle. Accordingly, the aforesaid predetermined range may be defined by the forward tilt angle θ1 and the backward tilt angle θ2. It should be noted that the aforesaid moving mode may include moving on a flat surface, moving upstairs or downstairs, or moving uphill or downhill. Furthermore, the forward tilt angle θ1 and the backward tilt angle θ2 mentioned in the above may be determined according to different users and different moving manners, wherein the forward tilt angle θ1 and the backward tilt angle θ2 may be identical or different. For example, the forward tilt angle and the backward tilt angle for moving upstairs or downstairs and moving uphill or downhill are larger than the forward tilt angle and the backward tilt angle for moving on the flat surface.

Accordingly, when the processing unit 128 determines that the tilt angle of the user exceeds the predetermined range defined by the forward tilt angle θ1 and the backward tilt angle θ2, and the tilt angle is relatively large, it means that the user is quite possible to fall down. At this time, the processing unit 128 may control the first driving unit 124 to increase the rotating speed of the rotating member 122 as the tilt angle of the user increases, so as to increase the moment of inertia M generated by the rotating member 122. On the other hand, when the tilt angle exceeds the predetermined range defined by the forward tilt angle θ1 and the backward tilt angle θ2, and the tilt angle is relatively small, it means that the user has a low risk of falling down. At this time, the processing unit 128 may control the first driving unit 124 to reduce the rotating speed of the rotating member 122 as the tilt angle of the user reduces, so as to reduce the moment of inertia M generated by the rotating member 122. Furthermore, when the processing unit 128 determines that the tilt angle of the user does not exceed the predetermined range, the processing unit 128 does not adjust the rotating speed of the rotating member 122 (step S184 in FIG. 4). It should be noted that, in an embodiment of the disclosure, when the tilt angle of the user exceeds the predetermined range defined by the forward tilt angle θ1 and the backward tilt angle θ2, the rotating speed of the rotating member 122 may vary linearly in accordance with the tilt angle of the user. For example, the larger the tilt angle is, the faster the rotating speed of the rotating member 122 is; and the smaller the tilt angle is, the slower the rotating speed of the rotating member 122 is.

After adjusting or not adjusting the rotating speed of the rotating member 122, the processing unit 128 further determines whether the current behavior mode has been converted into a standing posture (step S186 in FIG. 4). For example, in an embodiment, the disclosure may determine whether the behavior mode has been converted into the standing posture by determining whether an angle of a hip joint and a knee joint of the wearable device 1 are respectively equivalent to a predetermined angle of the hip joint and a predetermined angle of the knee joint of the standing posture. When the processing unit 128 determines that the current behavior mode has been converted into the standing posture, the processing unit 128 determines the current behavior mode from the plurality of behavior modes according to the sensing signal again (step S16 in FIG. 4). On the other hand, when the processing unit 128 determines that the current behavior mode has not been converted into the standing posture yet, the processing unit 128 determines whether the tilt angle sensed by the inertial measurement unit 1260 exceeds the predetermined range again (step S180 in FIG. 4), so as to determine whether to adjust the rotating speed of the rotating member 122 (steps S182 and S184 in FIG. 4).

When the current behavior mode is the sitting-to-standing mode (Step S20 in FIG. 4), the body of the user needs to move forward by a wide margin to stand up. At this time, the processing unit 128 controls the first driving unit 124 to adjust the rotating speed of the rotating member 122 to a predetermined value (step S200 in FIG. 4). In this embodiment, the predetermined value is smaller than the aforesaid predetermined initial rotating speed and the predetermined value may be set according to practical applications. In other words, when the current behavior mode is the sitting-to-standing mode, the processing unit 128 controls the first driving unit 124 to reduce the rotating speed of the rotating member 122 from the predetermined initial rotating speed to the predetermined value, so as to reduce the moment of inertia M generated by the rotating member 122. Accordingly, the disclosure may prevent the rotating member 122 from generating too large moment of inertia M to affect the motion of the user from sitting to standing.

After adjusting the rotating speed of the rotating member 122 to the predetermined value, the processing unit 128 further determines whether the current behavior mode has been converted into the standing posture (step S202 in FIG. 4). When the processing unit 128 determines that the current behavior mode has been converted into the standing posture, the processing unit 128 determines the current behavior mode from the plurality of behavior modes according to the sensing signal again (step S16 in FIG. 4). On the other hand, when the processing unit 128 determines that the current behavior mode has not been converted into the standing posture yet, the processing unit 128 keeps controlling the first driving unit 124 to adjust the rotating speed of the rotating member 122 to the predetermined value (step S200 in FIG. 4).

When the current behavior mode is the standing-to-sitting mode (step S22 in FIG. 4), the user has a low risk of falling down. That is to say, the user may not need assistance of the balance assistance system 12 from standing to sitting. At this time, the processing unit 128 controls the first driving unit 124 to reduce the rotating speed of the rotating member 122 gradually (step S220 in FIG. 4), so as to save power.

While reducing the rotating speed of the rotating member 122, the processing unit 128 continuously determines whether the current behavior mode has been converted into a sitting posture (step S222 in FIG. 4). For example, in an embodiment, the disclosure may determine whether the behavior mode has been converted into the sitting posture by determining whether the angle of the hip joint and the angle of the knee joint of the wearable device 1 are respectively equivalent to a predetermined angle of the hip joint and a predetermined angle of the knee join of the sitting posture. When the processing unit 128 determines that the current behavior mode has been converted into the sitting posture, the processing unit 128 may switch off the first driving unit 124 (step S224 in FIG. 4) to save power. Then, the processing unit 128 determines the current behavior mode from the plurality of behavior modes according to the sensing signal again (step S16 in FIG. 4). On the other hand, when the processing unit 128 determines that the current behavior mode has not been converted into the sitting posture yet, the processing unit 128 keeps controlling the first driving unit 124 to reduce the rotating speed of the rotating member 122 gradually (step S220 in FIG. 4).

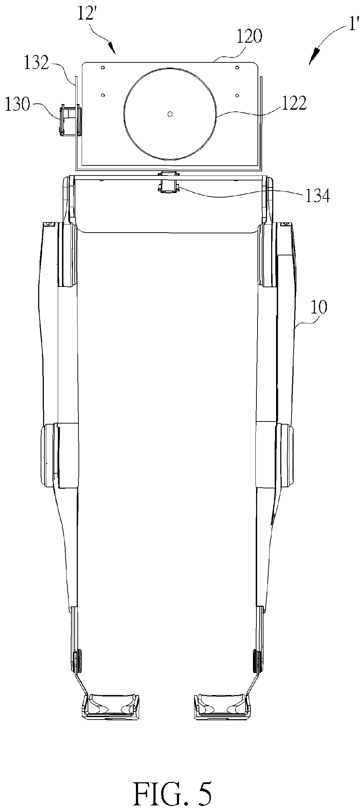

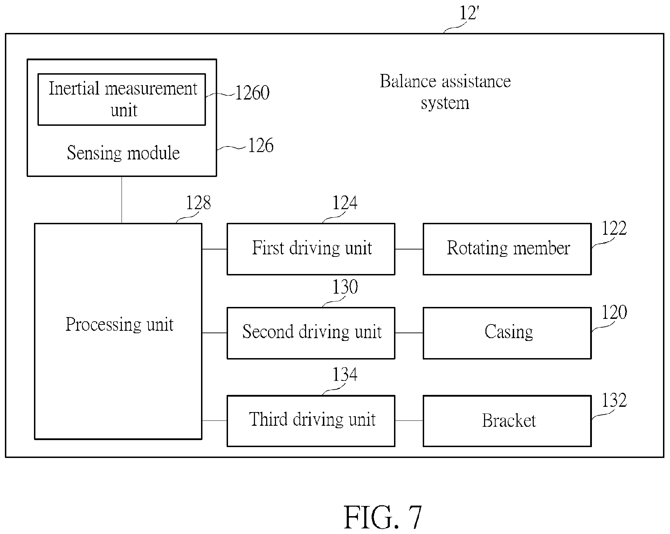

As shown in FIGs. 5 to 7, a balance assistance system 12' of a wearable device 1' of another embodiment may further include a second driving unit 130, a bracket 132, and a third driving unit 134. The casing 120 may be pivotally connected to the bracket 132. The second driving unit 130 may be disposed on the bracket 132 and connected to the casing 120. The second driving unit 130 is configured to drive the casing 120 to rotate. The third driving unit 134 may be disposed on the wearable object 10 and connected to the bracket 132. The third driving unit 134 is configured to drive the bracket 132 to rotate. Furthermore, the processing unit 128 is also electrically connected to the second driving unit 130 and the third driving unit 134. In this embodiment, the second driving unit 130 and the third driving unit 134 may be motors.

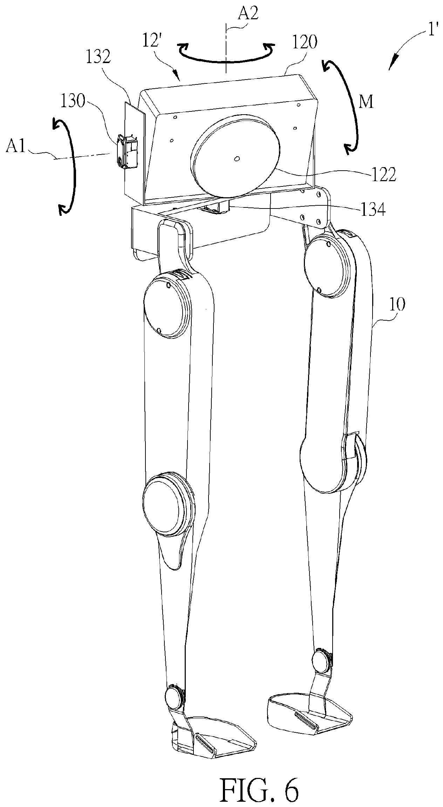

As shown in FIG. 6, the second driving unit 130 may drive the casing 120 to rotate with respect to a first axis A1 and the third driving unit 134 may drive the bracket 132 to rotate with respect to a second axis A2, wherein the first axis A1 is perpendicular to the second axis A2. Accordingly, the balance assistance system 12' of the wearable device 1' may change a direction of the moment of inertia M generated by the rotating member 122, so as to provide balance assistance for the user in any direction. For example, once the user is almost to fall down, the second driving unit 130 may drive the casing 120 to rotate and the third driving unit 134 may drive the bracket 132 to rotate to make the rotating shaft of the rotating member 122 parallel to a falling direction of the user, so as to provide the moment of inertia M for the user more precisely.

As mentioned in the above, the balance assistance system of the disclosure may be disposed on the wearable object (e.g. exoskeleton). When the wearable object is worn by a user and the balance assistance system is powered on, the first driving unit drives the rotating member to rotate to generate a moment of inertia. The moment of inertia generated by the rotating member may assist the user in balance to prevent the user from falling down. Furthermore, the sensing module generates the sensing signal according to a motion of the user. The balance assistance system of the disclosure may determine the current behavior mode of the user according to the sensing signal and adjust the rotating speed of the rotating member according to the current behavior mode of the user. Accordingly, the balance assistance system of the disclosure may flexibly adjust the moment of inertia generated by the rotating member according to different behavior modes of the user, so as to assist the user in balance in good time and avoid affecting normal motion of the user.

## Claims

1. A balance assistance system (12, 12') comprising:
a rotating member (122);
a first driving unit (124) connected to the rotating member (122), the first driving unit (124) being configured to drive the rotating member (122) to rotate;
a sensing module (126); and
a processing unit (128) **characterized in that** it is electrically connected to the first driving unit (124) and the sensing module (126), the processing unit (128) receiving a sensing signal from the sensing module (126), the processing unit (128) determining a current behavior mode of a human from a plurality of behavior modes of the human according to the sensing signal, the plurality of behavior modes comprising a moving mode, a sitting-to-standing mode and a standing-to-sitting mode, the processing unit (128) controlling the first driving unit (124) to adjust a rotating speed of the rotating member (122) according to the current behavior mode.

2. The balance assistance system (12, 12') of claim 1 further **characterized in that** the sensing module (126) comprises an inertial measurement unit (1260); when the current behavior mode is the moving mode, the processing unit (128) determines whether a tilt angle sensed by the inertial measurement unit (1260) exceeds a predetermined range; when the processing unit (128) determines that the tilt angle exceeds the predetermined range, the processing unit (128) controls the first driving unit (124) to adjust the rotating speed of the rotating member (122) according to a variation of the tilt angle; after adjusting the rotating speed of the rotating member (122), the processing unit (128) determines whether the current behavior mode has been converted into a standing posture; when the processing unit (128) determines that the current behavior mode has been converted into the standing posture, the processing unit (128) determines the current behavior mode from the plurality of behavior modes according to the sensing signal again.

3. The balance assistance system (12, 12') of claim 1 further **characterized in that** when the current behavior mode is the sitting-to-standing mode, the processing unit (128) controls the first driving unit (124) to adjust the rotating speed of the rotating member (122) to a predetermined value; after adjusting the rotating speed of the rotating member (122) to the predetermined value, the processing unit (128) determines whether the current behavior mode has been converted into a standing posture; when the processing unit (128) determines that the current behavior mode has been converted into the standing posture, the processing unit (128) determines the current behavior mode from the plurality of behavior modes according to the sensing signal again.

4. The balance assistance system (12, 12') of claim 1 further **characterized in that** when the current behavior mode is the standing-to-sitting mode, the processing unit (128) controls the first driving unit (124) to reduce the rotating speed of the rotating member (122) gradually; while reducing the rotating speed of the rotating member, the processing unit (128) determines whether the current behavior mode has been converted into a sitting posture; when the processing unit (128) determines that the current behavior mode has been converted into the sitting posture, the processing unit (128) switches off the first driving unit (124) and determines the current behavior mode from the plurality of behavior modes according to the sensing signal again.

5. The balance assistance system (12, 12') of claim 1 further **characterized in that** when the balance assistance system is powered on, the processing unit (128) controls the first driving unit (124) to drive the rotating member (122) to rotate by a predetermined initial rotating speed.

6. The balance assistance system (12') of claim 1 further **characterized in that** the balance assistance system (12') further comprises a casing (120) and a second driving unit (130), the rotating member (122) is disposed on the casing (120), the first driving unit (124) is disposed in the casing (120), the second driving unit (130) is connected to the casing (120), the processing unit (128) is electrically connected to the second driving unit (130), and the second driving unit (130) is configured to drive the casing (120) to rotate with respect to a first axis (A1).

7. The balance assistance system (12') of claim 6 further **characterized in that** the balance assistance system (12') further comprises a bracket (132) and a third driving unit (134), the casing (120) is pivotally connected to the bracket (132), the third driving unit (134) is connected to the bracket (132), the processing unit (128) is electrically connected to the third driving unit (134), the third driving unit (134) is configured to drive the bracket (132) to rotate with respect to a second axis (A2), and the first axis (A1) is perpendicular to the second axis (A2).

8. A wearable device (1, 1') **characterized by** the wearable device (1, 1') comprising:
a wearable object (10); and
the balance assistance system (12, 12') of any of claims 1 to 7 disposed on the wearable object (10).

## Patentansprüche

1. Gleichgewichtsunterstützungssystem (12, 12'), welches umfasst:
ein Drehelement (122);
eine erste Antriebseinheit (124), die mit dem Drehelement (122) verbunden ist, worin die erste Antriebseinheit (124) ausgestaltet ist, das Drehelement (122) zum Drehen anzutreiben;
ein Sensormodul (126); und
eine Verarbeitungseinheit (128)
**dadurch gekennzeichnet, dass** diese elektrisch mit der ersten Antriebseinheit (124) und dem Sensormodul (126) verbunden ist, wobei die Verarbeitungseinheit (128) ein Erfassungssignal von dem Sensormodul (126) empfängt, wobei die Verarbeitungseinheit (128) einen aktuellen Verhaltensmodus eines Menschen aus mehreren Verhaltensmodi des Menschen gemäß dem Erfassungssignal bestimmt, worin die mehreren Verhaltensmodi einen Bewegungsmodus, einen Modus vom Sitzen zum Stehen und einen Modus vom Stehen zum Sitzen umfassen, wobei die Verarbeitungseinheit (128) die erste Antriebseinheit (124) steuert, eine Drehgeschwindigkeit des Drehelements (122) gemäß dem aktuellen Verhaltensmodus anzupassen.

2. Gleichgewichtsunterstützungssystem (12, 12') nach Anspruch 1, welches ferner **dadurch gekennzeichnet ist, dass** das Sensormodul (126) eine Trägheitsmesseinheit (1260) umfasst; wobei, wenn der aktuelle Verhaltensmodus der Bewegungsmodus ist, die Verarbeitungseinheit (128) bestimmt, ob ein von der Trägheitsmesseinheit (1260) erfasster Neigungswinkel einen bestimmten Bereich überschreitet; wobei, wenn die Verarbeitungseinheit (128) bestimmt, dass der Neigungswinkel den bestimmten Bereich überschreitet, die Verarbeitungseinheit (128) die erste Antriebseinheit (124) steuert, die Drehgeschwindigkeit des Drehelements (122) entsprechend einer Änderung des Neigungswinkels anzupassen; wobei nach dem Einstellen der Drehgeschwindigkeit des Drehelements (122) die Verarbeitungseinheit (128) bestimmt, ob der aktuelle Verhaltensmodus in eine stehende Haltung umgewandelt worden ist; wobei, wenn die Verarbeitungseinheit (128) bestimmt, dass der aktuelle Verhaltensmodus in die stehende Haltung umgewandelt worden ist, die Verarbeitungseinheit (128) den aktuellen Verhaltensmodus aus den mehreren Verhaltensmodi gemäß dem Erfassungssignal erneut bestimmt.

3. Gleichgewichtsunterstützungssystem (12, 12') nach Anspruch 1, **dadurch gekennzeichnet, dass** die Verarbeitungseinheit (128) die erste Antriebseinheit (124) steuert, die Drehgeschwindigkeit des Drehelements (122) auf einen bestimmten Wert einzustellen, wenn der aktuelle Verhaltensmodus der Modus vom Sitzen zum Stehen ist; und darin dass nach dem Einstellen der Drehgeschwindigkeit des Drehelements (122) auf den bestimmten Wert die Verarbeitungseinheit (128) bestimmt, ob der aktuelle Verhaltensmodus in eine stehende Haltung umgewandelt worden ist; wobei, wenn die Verarbeitungseinheit (128) bestimmt, dass der aktuelle Verhaltensmodus in die stehende Haltung umgewandelt worden ist, die Verarbeitungseinheit (128) den aktuellen Verhaltensmodus aus den mehreren Verhaltensmodi gemäß dem Erfassungssignal erneut bestimmt.

4. Gleichgewichtsunterstützungssystem (12, 12') nach Anspruch 1, **dadurch gekennzeichnet, dass**, wenn der aktuelle Verhaltensmodus der Modus vom Stehen zum Sitzen ist, die Verarbeitungseinheit (128) die erste Antriebseinheit (124) steuert, die Drehgeschwindigkeit des Drehelements (122) allmählich zu reduzieren; wobei während die Drehgeschwindigkeit des Drehelements reduziert wird, die Verarbeitungseinheit (128) bestimmt, ob der aktuelle Verhaltensmodus in eine sitzende Haltung umgewandelt wurde; wobei, wenn die Verarbeitungseinheit (128) feststellt, dass der aktuelle Verhaltensmodus in die sitzende Haltung umgewandelt worden ist, die Verarbeitungseinheit (128) die erste Antriebseinheit (124) ausschaltet und den aktuellen Verhaltensmodus aus den mehreren Verhaltensmodi gemäß dem Erfassungssignal erneut bestimmt.

5. Gleichgewichtsunterstützungssystem (12, 12') nach Anspruch 1, **dadurch gekennzeichnet, dass** die Verarbeitungseinheit (128) beim Einschalten des Gleichgewichtsunterstützungssystems die erste Antriebseinheit (124) steuert, das Drehelement (122) mit einer bestimmten Anfangsdrehzahl zu drehen.

6. Gleichgewichtsunterstützungssystem (12') nach Anspruch 1 welches ferner **dadurch gekennzeichnet ist, dass** das Gleichgewichtsunterstützungssystem (12') zudem ein Gehäuse (120) und eine zweite Antriebseinheit (130) umfasst, worin das Drehelement (122) an dem Gehäuse (120) angeordnet ist, die erste Antriebseinheit (124) in dem Gehäuse (120) angeordnet ist, die zweite Antriebseinheit (130) mit dem Gehäuse (120) verbunden ist, die Verarbeitungseinheit (128) mit der zweiten Antriebseinheit (130) elektrisch verbunden ist, und die zweite Antriebseinheit (130) ausgestaltet ist, das Gehäuse (120) anzutreiben, sich in Bezug auf eine erste Achse (A1) zu drehen.

7. Gleichgewichtsunterstützungssystem (12') nach Anspruch 6 welches ferner **dadurch gekennzeichnet ist, dass** das Gleichgewichtsunterstützungssystem (12') zudem eine Halterung (132) und eine dritte Antriebseinheit (134) umfasst, worin das Gehäuse (120) schwenkbar mit der Halterung (132) verbunden ist, die dritte Antriebseinheit (134) mit der Halterung (132) verbunden ist, die Verarbeitungseinheit (128) mit der dritten Antriebseinheit (134) elektrisch verbunden ist, die dritte Antriebseinheit (134) ausgestaltet ist, die Halterung (132) anzutreiben, sich in Bezug auf eine zweite Achse (A2) zu drehen, und die erste Achse (A1) senkrecht zu der zweiten Achse (A2) ist.

8. Tragbare Vorrichtung (1, 1'), **dadurch gekennzeichnet, dass** die tragbare Vorrichtung (1, 1') umfasst:
einen tragbaren Gegenstand (10); und
das Gleichgewichtsunterstützungssystem (12, 12') nach einem der Ansprüche 1 bis 7, das an dem tragbaren Gegenstand (10) angeordnet ist.

## Revendications

1. Système d'assistance à l'équilibre (12, 12') comprenant:
un élément rotatif (122);
une première unité d'entraînement (124) reliée à l'élément rotatif (122), la première unité d'entraînement (124) étant configurée pour entraîner l'élément rotatif (122) en rotation;
un module de détection (126); et
une unité de traitement (128)
**caractérisé en ce qu'**il est connecté électriquement à la première unité d'entraînement (124) et au module de détection (126), l'unité de traitement (128) recevant un signal de détection du module de détection (126), l'unité de traitement (128) déterminant un mode de comportement actuel d'un être humain à partir d'une pluralité de modes de comportement de l'être humain en fonction du signal de détection, la pluralité de modes de comportement comprenant un mode de déplacement, un mode de passage de la position assise à la position debout et un mode de passage de la position debout à la position assise, l'unité de traitement (128) commandant la première unité d'entraînement (124) pour ajuster une vitesse de rotation de l'élément rotatif (122) en fonction du mode de comportement actuel.

2. Système d'assistance à l'équilibre (12, 12') de la revendication 1, **caractérisé en outre en ce que** le module de détection (126) comprend une unité de mesure inertielle (1260); lorsque le mode de comportement actuel est le mode de déplacement, l'unité de traitement (128) détermine si un angle d'inclinaison détecté par l'unité de mesure inertielle (1260) dépasse une plage prédéterminée; lorsque l'unité de traitement (128) détermine que l'angle d'inclinaison dépasse la plage prédéterminée, l'unité de traitement (128) commande la première unité d'entraînement (124) pour ajuster la vitesse de rotation de l'élément rotatif (122) en fonction d'une variation de l'angle d'inclinaison; après avoir ajusté la vitesse de rotation de l'élément rotatif (122), l'unité de traitement (128) détermine si le mode de comportement actuel a été converti en une posture debout; lorsque l'unité de traitement (128) détermine que le mode de comportement actuel a été converti en posture debout, l'unité de traitement (128) détermine à nouveau le mode de comportement actuel parmi la pluralité de modes de comportement en fonction du signal de détection.

3. Système d'assistance à l'équilibre (12, 12') de la revendication 1, **caractérisé en outre en ce que** lorsque le mode de comportement actuel est le mode de passage de la position assise à la position debout, l'unité de traitement (128) commande la première unité d'entraînement (124) pour ajuster la vitesse de rotation de l'élément rotatif (122) à une valeur prédéterminée; après avoir ajusté la vitesse de rotation de l'élément rotatif (122) à la valeur prédéterminée, l'unité de traitement (128) détermine si le mode de comportement actuel a été converti en une posture debout; lorsque l'unité de traitement (128) détermine que le mode de comportement actuel a été converti en une posture debout, l'unité de traitement (128) détermine à nouveau le mode de comportement actuel parmi la pluralité de modes de comportement en fonction du signal de détection.

4. Système d'assistance à l'équilibre (12, 12') de la revendication 1, **caractérisé en outre en ce que** lorsque le mode de comportement actuel est le mode de passage de la position debout à la position assise, l'unité de traitement (128) commande la première unité d'entraînement (124) pour réduire progressivement la vitesse de rotation de l'élément rotatif (122); tout en réduisant la vitesse de rotation de l'élément rotatif, l'unité de traitement (128) détermine si le mode de comportement actuel a été converti en une posture assise; lorsque l'unité de traitement (128) détermine que le mode de comportement actuel a été converti en une posture assise, l'unité de traitement (128) éteint la première unité d'entraînement (124) et détermine à nouveau le mode de comportement actuel parmi la pluralité de modes de comportement en fonction du signal de détection.

5. Système d'assistance à l'équilibre (12, 12') de la revendication 1, **caractérisé en outre en ce que**, lorsque le système d'assistance à l'équilibre est mis sous tension, l'unité de traitement (128) commande la première unité d'entraînement (124) pour entraîner l'élément rotatif (122) en rotation à une vitesse de rotation initiale prédéterminée.

6. Système d'assistance à l'équilibre (12') de la revendication 1, **caractérisé en ce que** le système d'assistance à l'équilibre (12') comprend en outre un boîtier (120) et une seconde unité d'entraînement (130), l'élément rotatif (122) est disposé sur le boîtier (120), la première unité d'entraînement (124) est disposée dans le boîtier (120), la seconde unité d'entraînement (130) est connectée au boîtier (120), l'unité de traitement (128) est connectée électriquement à la seconde unité d'entraînement (130), et la seconde unité d'entraînement (130) est configurée pour entraîner le boîtier (120) en rotation par rapport à un premier axe (A1).

7. Système d'assistance à l'équilibre (12') de la revendication 6, **caractérisé en ce que** le système d'assistance à l'équilibre (12') comprend en outre un support (132) et une troisième unité d'entraînement (134), le boîtier (120) est relié de manière pivotante au support (132), la troisième unité d'entraînement (134) est reliée au support (132), l'unité de traitement (128) est connectée électriquement à la troisième unité d'entraînement (134), la troisième unité d'entraînement (134) est configurée pour entraîner le support (132) en rotation par rapport à un deuxième axe (A2), et le premier axe (A1) est perpendiculaire au deuxième axe (A2).

8. Dispositif portable (1, 1') **caractérisé en ce que** le dispositif portable (1, 1') comprend:
un objet portable (10); et
le système d'assistance à l'équilibre (12, 12') de l'une quelconque des revendications 1 à 7 disposé sur l'objet portable (10).
